# FASCICULE DE BREVET EUROPEEN

(11) **EP 2 222 865 B1**
(45) Date de publication et mention de la délivrance du brevet: **04.05.2011**
(21) Numéro de dépôt: 08872168.3
(22) Date de dépôt: 06.11.2008
(51) Int. Cl.: C12P 7/10, C12N 9/42, C12F 3/10

(54) **Procédé de production d'alcool dans un contexte de bioraffinerie**
Verfahren zur Herstellung von Alkohol im Kontext einer Bioraffinerie
Method for producing alcohol in the context of a bio-refinery

(30) Priorité: 21.11.2007 FR 0708173
(43) Date de publication de la demande: 01.09.2010
(73) Titulaire: IFP Energies nouvelles, 92852 Rueil-Malmaison Cedex (FR)
(72) Inventeur: MARGEOT Antoine, F-75020 Paris (FR); MONOT Frédéric, F-92000 Nanterre (FR)
(86) Numéro de dépôt international: PCT/FR2008/001566
(87) Numéro de publication internationale: WO 2009/098365

(56) Documents cités:
- US-A- 4 326 036
- LAWFORD HUGH G ET AL: "Comparative ethanol productivities of different Zymomonas recombinants fermenting oat hull hydrolysate" APPLIED BIOCHEMISTRY AND BIOTECHNOLOGY, vol. 91-93, avril 2001 (2001-04), pages 133-146, XP002492567 ISSN: 0273-2289
- MISHRA S ET AL: "A CONSTITUTIVELY CELLULASE PRODUCING MUTANT OF TRICHODERMA-REESEI" BIOTECHNOLOGY AND BIOENGINEERING, vol. 24, no. 1, 1982, pages 251-254, XP002492688 ISSN: 0006-3592
- BOOPATHY R ET AL: "Microbial decomposition of post-harvest sugarcane residue" BIORESOURCE TECHNOLOGY, ELSEVIER, GB, vol. 79, no. 1, 1 août 2001 (2001-08-01), pages 29-33, XP002384342 ISSN: 0960-8524
- CAMASSOLA M ET AL: "Production of cellulases and hemicellulases by Penicillium echinulatum grown on pretreated sugar cane bagasse and wheat bran in solid-state fermentation" JOURNAL OF APPLIED MICROBIOLOGY, [Online] vol. 103, no. 6, 19 juin 2007 (2007-06-19), pages 2196-2204, XP002492568 ISSN: 1364-5072 [extrait le 2008-08-18]

## Description

### Domaine de l'invention

La présente invention s'inscrit dans le cadre de la production de biocarburants. Elle concerne plus particulièrement la production d'enzymes cellulolytiques et/ou hémicellulolytiques dans le cadre de la production d'alcool à partir de matériaux cellulosiques ou ligno-cellulosiques (biocarburant de seconde génération).

La logique économique et logistique actuelle veut que les sites de production de biocarburants de seconde génération soient les mêmes que ceux de production de première génération, l'ensemble constituant une "bioraffinerie" où l'intégralité du végétal est valorisée. Ainsi en partant d'une plante sucrière, on cherche à valoriser la canne à sucre et les résidus lignocellulosiques de canne.

### Étude de l'art antérieur

Depuis les années 1970, la transformation de la biomasse ligno-cellulosique en éthanol, après hydrolyse des polysaccharides constitutifs en sucres fermentescibles, a fait l'objet de nombreux travaux.

Les résidus lignocellulosiques sont constitués pour la plupart d'environ 40 à 50 % de cellulose, 20 à 25 % d'hémicellulose et de 15 à 25 % de lignine.

De ces trois polymères, cellulose, hémicellulose et lignine, la cellulose est la principale source de sucres fermentescibles en éthanol car elle est constituée de glucose, ce dernier étant facilement fermenté en éthanol par *Saccharomyces cerevisiae* dans des procédés industriels éprouvés et performants. Les pentoses contenus dans les hémicelluloses ne sont pas efficacement convertis en éthanol. D'autres microorganismes parmi les genres *Saccharomyces, Pichia, Candida, Pachysolen, Zymomonas, Klebsiella, Escherichia* peuvent être choisis pour valoriser les sucres monomères issus de la biomasse en éthanol.

Le procédé de transformation des matériaux ligno-cellulosiques en alcool comprend une étape de prétraitement physico-chimique, suivie d'une étape d'hydrolyse enzymatique ou chimique, d'une étape de fermentation alcoolique des sucres libérés et d'une étape de récupération de l'alcool.

L'étape de prétraitement a pour objet de libérer les sucres contenus dans les hémicelluloses sous forme de monomères, essentiellement des pentoses, comme le xylose et l'arabinose, et des hexoses, comme le galactose, le mannose et le glucose, et d'améliorer l'accessibilité de la cellulose engluée dans la matrice de lignine et hémicellulose. De nombreuses technologies existent : cuissons acides, cuissons alcalines, explosion à la vapeur, procédés organosolv, etc. L'efficacité du prétraitement se mesure par le taux de récupération des hémicelluloses et par la susceptibilité à l'hydrolyse du résidu cellulosique. Les prétraitements acides en conditions douces et par explosion à la vapeur sont les mieux adaptés. Ils permettent une récupération totale des pentoses et une bonne accessibilité de la cellulose à l'hydrolyse.

Le résidu cellulosique est hydrolysé, soit par voie acide, soit par voie enzymatique avec l'utilisation d'enzymes cellulolytiques et/ou hémicellulolytiques. Des microorganismes, comme les champignons appartenant aux genres *Trichoderma, Aspergillus, Penicillium ou Schizophyllum,* ou les bactéries anaérobies appartenant par exemple au genre *Clostridium,* produisent ces enzymes, contenant notamment les cellulases et les xylanases, adaptées à l'hydrolyse totale des polymères constituant les végétaux.

La voie acide, effectuée à l'aide d'acide fort, acide sulfurique notamment, est efficace mais requiert d'importantes quantités de produits chimiques (acide puis base pour la neutralisation). L'hydrolyse enzymatique ne présente pas cet inconvénient ; elle s'effectue par ailleurs dans des conditions douces et est efficace. Par contre, le coût des enzymes reste très élevé. De ce fait, beaucoup de travaux ont été conduits pour réduire ce coût : l'augmentation de la production d'enzymes d'abord, en sélectionnant les souches hyperproductrices et en améliorant les procédés de fermentation, la diminution de la quantité d'enzymes en hydrolyse ensuite, en optimisant la phase de prétraitement ou en améliorant l'activité spécifique de ces enzymes. Au cours de la dernière décennie, les principaux travaux se sont attachés à comprendre les mécanismes d'action des cellulases et d'expression des enzymes afin de faire excréter le complexe enzymatique le plus approprié à l'hydrolyse des substrats ligno-cellulosiques en modifiant les souches avec les outils de biologie moléculaire.

Le microorganisme le plus utilisé pour la production de cellulases est le champignon *Trichoderma reesei.* Les souches sauvages ont la faculté d'excréter, en présence d'un substrat inducteur, la cellulose par exemple, le complexe enzymatique considéré comme le mieux adapté à l'hydrolyse de la cellulose. Les enzymes du complexe enzymatique contiennent trois grands types d'activités : les endoglucanases, les exoglucanases et les cellobiases. D'autres protéines possédant des propriétés indispensables à l'hydrolyse des matériaux ligno-cellulosiques sont également produites par *Trichoderma reesei,* les xylanases par exemple. La présence d'un substrat inducteur est indispensable à l'expression des enzymes cellulolytiques et/ou hémicellulolytiques. La nature du substrat carboné a une forte influence sur la composition du complexe enzymatique. C'est le cas du xylose, qui, associé à un substrat carboné inducteur comme la cellulose ou le lactose, permet d'améliorer significativement l'activité dite xylanase.

Les techniques de génétique classique par mutation ont permis la sélection de souches de *Trichoderma reesei* hyperproductrices de cellulases telles que les souches MCG77 (Gallo - brevet US 4275 167), MCG 80 (Allen, A.L. et Andreotti, R.E., Biotechnol-Bioengi 1982; 12, 451-459 1982), RUT C30 (Montenecourt, B.S. et Eveleigh, D.E., Appl. Environ. Microbiol. 1977, 34, 777-782) et CL847 (Durand et al, 1984, Proc. Colloque SFM "Génétique des microorganismes industriels". Paris. H. HESLOT Ed, pp 39-50). Les améliorations ont permis d'obtenir des souches hyperproductrices, moins sensibles à la répression catabolique sur sucres monomères notamment, glucose par exemple, par rapport aux souches sauvages.

Des souches recombinantes ont été obtenues à partir des souches de *Trichoderma reesei* Qm9414, RutC30, CL847 en clonant des gènes hétérologues, par exemple l'invertase *d'Aspergillus niger* permettant à *Trichoderma reesei* d'utiliser le saccharose comme source de carbone. Ces souches ont conservé leur hyperproductivité et leur aptitude à être cultivées en fermenteur.

Le procédé de production de cellulases par *Trichoderma reesei* a fait l'objet d'améliorations importantes en vue de l'extrapolation à l'échelle industrielle.

Pour obtenir de bonnes productivités en enzymes, il est nécessaire d'apporter une source de carbone rapidement assimilable pour la croissance de *Trichoderma reesei* et un substrat inducteur qui permette l'expression des cellulases et la sécrétion dans le milieu de culture. La cellulose peut jouer ces deux rôles ; cependant, elle est difficile d'utilisation au stade industriel et elle a été remplacée par des sources de carbone solubles comme le glucose, le xylose ou le lactose, le lactose jouant également le rôle de substrat inducteur. D'autres sucres solubles comme le cellobiose et le sophorose ont été décrits comme inducteurs, mais ils sont trop onéreux pour être utilisés au stade industriel. On a cependant constaté que les productions de cellulases par *Trichoderma reesei,* avec des substrats solubles, sont très inférieures à celles obtenues sur cellulose en "batch". Ceci est dû à l'effet répresseur des sucres facilement assimilables, à forte concentration. L'alimentation en continu des substrats carbonés solubles a permis de lever la répression catabolique en limitant la concentration résiduelle dans les cultures et en optimisant la quantité de sucre permettant d'obtenir un meilleur rendement et une meilleure productivité enzymatique (voir le brevet FR-B-2 555 603 déposé au nom de la Demanderesse).

Le lactose reste, dans un procédé de production industriel d'enzymes cellulolytiques, un des substrats les plus appropriés et l'un des moins chers ; il reste néanmoins coûteux et représente environ un tiers du prix de revient des enzymes. Malgré tous les progrès réalisés, le coût des enzymes reste un poste important (de 30 à 50 %) dans la transformation de la biomasse cellulosique en alcool. De plus, dans le cas de l'utilisation de lactose comme source de carbone pour la production de cellulases, le procédé est dépendant d'une source de carbone extérieure. De ce fait, l'utilisation de substrats carbonés issus de la filière (comme des résidus d'hémicelluloses hydrolysées ou des vinasses de fermentation éthanoliques) est une voie de progrès importante si la source de carbone inductrice est facilement disponible.

L'utilisation des résidus d'hémicelluloses hydrolysés pour la production de cellulases a été décrite dans la demande de brevet FR-A-2 881 753 déposée au nom de la Demanderesse. Ces résidus sont effectivement inducteurs de la production de cellulases et conduisent à des mélanges efficaces en hydrolyse de matériaux lignocellulosiques. L'utilisation de ces résidus présente néanmoins un certain nombre de limitations potentielles:
- leur utilisation impose un prétraitement où seront solubilisées les hémicelluloses.
- une variation de matière première végétale comme par exemple un simple changement de variété risque d'entraîner une modification dans la composition du résidu, et par là même une modification de la composition en enzymes.
- la présence d'inhibiteurs, notamment en fonction du végétal et des conditions de prétraitement, risque de poser des problèmes dans la reproductibilité des cultures
- la forte teneur en xylose des résidus (issus de paille par exemple) induit des hémicellulases qui ne sont pas forcément requises pour hydrolyser un substrat déjà débarrassé de la grande majorité de ses hémicelluloses.

D'autre part, l'utilisation de vinasses de fermentation d'hydrolysats de matière lignocellulosique a été également décrite dans cette demande de brevet. Les performances obtenues avec cette source de carbone sont équivalentes voire supérieures à celles obtenues avec les substrats classiques, mais il est possible que les nouvelles générations d'enzymes aux propriétés d'hydrolyse améliorées, et les souches de levures adaptées aux conditions de fermentation ne réduisent la quantité de source de carbone inductrice dans une proportion incompatible avec une production d'enzyme.

Le document Lawford H. G. et al. (Applied Biochemistry and Biotechnology, 2001, vol. 91-93, pages 133-146) décrit des tests comparatifs réalisés pour la productivité d'éthanol à partir de biomasse utilisant différents recombinants d'une souche *Zymomonas mobilis* utilisée pour l'étape de fermentation éthanolique, cette souche étant capable de co-fermenter le glucose et le xylose qui sont contenus dans les hydrolysats de biomasse. Les hydrolysats utilisés sont préparés à partir de la paille d'avoine prétraitée par explosion à la vapeur, suivi par l'hydrolyse avec des cellulases. Dans ce document, il est fait référence au procédé de l'entreprise logen (Canada) qui utilise le même type d'hydrolysat. Il est mentionné qu'un site de production de bioéthanol de l'entreprise logen est co-localisé avec un site de production d'enzymes, ce qui présente l'avantage que l'enzyme peut être utilisée sans le besoin de stabilisation et de préservation et que les sucres produits dans le procédé peuvent être utilisés pour la production des enzymes.

L'utilisation d'une source de carbone de composition reproductible, dépourvue d'inhibiteurs, et ne dépendant pas ou peu de la matière première à traiter serait une voie de progrès importante. Cette source de carbone, en restant disponible sur site, permet également de développer le concept de bioraffinerie.

### Résumé de l'invention

La présente invention décrit un procédé de production d'alcool à partir de biomasse lignocellulosique prétraitée dans lequel l'étape d'hydrolyse enzymatique est réalisée avec des enzymes cellulolytiques et/ou hémicellulolytiques produites en utilisant au moins un effluent issu d'un autre procédé de production d'éthanol utilisant comme matière première une plante sucrière, ledit effluent comprenant des jus sucrés issus des lavages de betteraves ou de cannes à sucre, et/ou des mélasses de sucrerie, la production d'enzymes étant réalisée avec des souches de microorganisme cellulolytique appartenant aux genres *Trichoderma, Aspergillus, Penicillum ou Schizophyllumen,* utilisant le saccharose comme source de carbone pour sa croissance, naturellement ou après modification.

### Description des figures

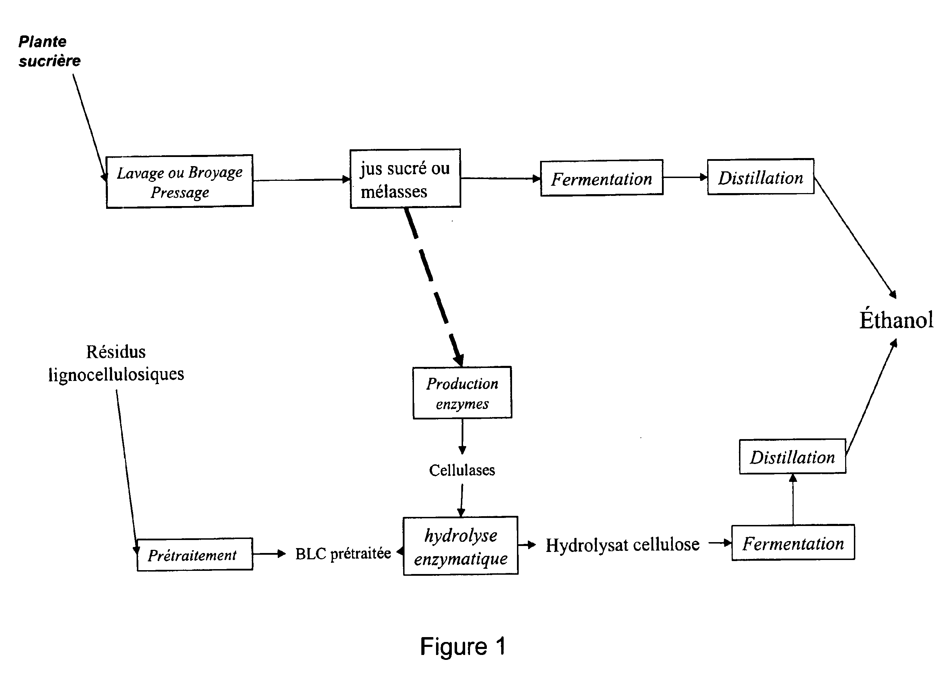

La Figure 1 décrit un schéma de procédé de transformation de production d'alcool selon la présente invention.
La Figure 2 décrit un schéma de procédé de transformation de production d'alcool selon la présente invention utilisant comme matière première lignocellulosique les résidus d'une plante sucrière.

### Description détaillée de l'invention

La présente invention concerne l'utilisation d'effluents obtenus au cours d'autres procédés de production de biocarburants, par exemple mais de façon non exclusive, les jus sucrés issus de lavages de betterave ou de cannes à sucres, ou mélasses de sucreries pour la production d'enzymes cellulolytiques et/ou hémicellulolytiques, avec des souches de microorganisme cellulolytique utilisant le saccharose comme source de carbone, naturellement ou après modification.

Le microorganisme cellulolytique appartient aux genres *Trichoderma, Aspergillus, Penicillium* ou *Schizophyllum* et préférentiellement à l'espèce *Trichoderma reesei.*

Les jus sucrés contenant le saccharose utilisé dans la présente invention proviennent de procédés de production de biocarburants de première génération et sont issus du lavage de betteraves ou de cannes à sucre, ou proviennent des mélasses de sucreries.

La source de carbone principale peut être le saccharose issu de ces jus, ou constituer un complément à un sucre soluble industriel, comme le lactose ou le xylose, ou un extrait de la fraction hémicellulosique sous forme de monomères provenant de la biomasse prétraitée, ou un extrait de vinasses de fermentation de produits d'hydrolyse de biomasse lignocellulosique, ou un mélange de ceux-ci. Ces deux derniers extraits peuvent également servir comme inducteurs de la production de cellulases.

Un objet de l'invention est de proposer une source de carbone facilement disponible, permettant de produire des enzymes cellulolytiques et/ou hémicellulolytiques aux activités appropriées à l'hydrolyse de la biomasse cellulosique, compatible avec plusieurs schémas de procédés de production de bioalcool dans différents contextes.

La source de carbone pour la croissance, principalement du saccharose, est obtenue à partir de jus sucrés suite au lavage par diffusion de pulpes de betteraves, ou à partir de mélasse suite à la raffinerie du sucre. De même, des jus sucrés ou mélasses issues de lavage de cannes à sucres peuvent être utilisés.

L'étape spécifique de la préparation du moût à partir de plantes saccharifères est l'extraction du saccharose. Ce sucre se trouve à l'état libre dans les vacuoles des cellules du végétal. Son extraction se fait soit par un pressage, soit par un lavage à l'eau chaude, pratiqué à contre-courant sur le végétal préalablement découpé.

Ainsi, les betteraves sont généralement lavées pour les débarrasser de la terre, puis découpées en cossettes. Un jus sucré est ensuite extrait par diffusion à l'eau chaude. Ce premier jus est habituellement destiné à fermentation éthanolique mais peut être utilisé, concentré ou non sous forme de sirop, dans la présente invention comme source de carbone pour la production de cellulases. Les mélasses, sous-produits du raffinage du sucre, contenant jusqu'à 50% en poids de saccharose, peuvent être également utilisées (Table 2).

Dans le cas de la canne à sucre, les cannes sont broyées et pressées avec ajout d'eau chaude pour solubiliser les sucres. Le jus sucré, éventuellement concentré, suit ensuite une voie similaire à celle de la voie betterave.

**Table 1 : Composition à titre indicatif des jus sucrés issus de la diffusion à l'eau chaude des betteraves et du broyage et pressage des cannes à sucre. La proportion d'impuretés est généralement réduite par filtration et/ou précipitation des sels.**

| | Jus sucré de betterave | Jus sucré de canne à sucre |
|---|---|---|
| Matière sèche (% poids) | 15 | 20 |
| Sucres totaux (%MS) | 94 | >90 |
| dont Saccharose (%MS) | 94 | 85 |
| dont Glucose (%MS) | 0 | 7.5 |
| dont Fructose (%MS) | 0 | 7.5 |
| Autres (%MS) | 6 | <10 |

**Table 2 : composition chimique à titre indicatif de mélasses de betterave et de canne à sucre (sources : INRA et Yang et al. (2007))**

| | Mélasse de Betterave | Mélasse de Canne à Sucre |
|---|---|---|
| Matière sèche (% poids) | 73 | 73 |
| Matières minérales (%MS) | 13 | 14 |
| Matière azotées totales (%MS) | 15 | 6 |
| Sucres totaux (%MS) | 64 | 64 |
| dont Saccharose (%MS) | 64 | 43 |
| dont Glucose (%MS) | 0 | 10.5 |
| dont Fructose (%MS) | 0 | 10.5 |
| Calcium (g/kg MS) | 3.7 | 7.4 |
| Phosphore (g/kg MS) | 0.3 | 0.7 |
| Potassium (g/kg MS) | 82 | 40 |

La biomasse lignocellulosique utilisée est choisie parmi les pailles, le bois, les cultures forestières, les résidus de plantes alcooligènes, sucrières et céréalières, les résidus de l'industrie papetière et les produits de transformation des matériaux cellulosiques et lignocellulosiques.

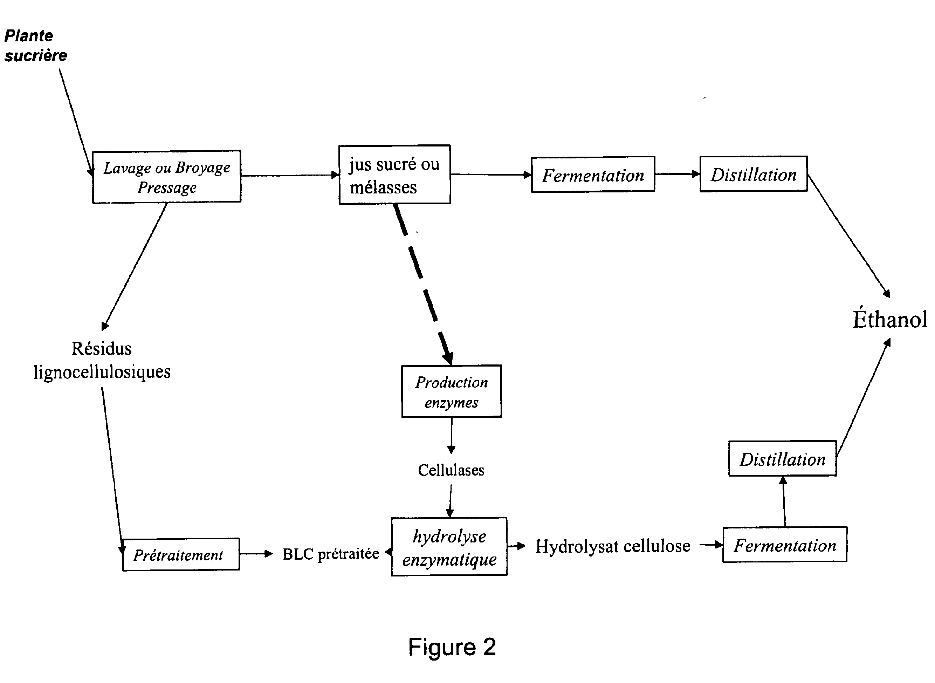

Dans le mode de réalisation représenté sur la Figure 2, les résidus lignocellulosiques proviennent de la plante sucrière à partir de laquelle les jus sucrés utilisés pour la production d'enzymes cellulolytiques et/ou hémicellulolytiques sont obtenus.

La matière première lignocellulosique subit un prétraitement qui peut être de toute nature, avec hydrolyse ou non des hémicelluloses. Après prétraitement, la fraction cellulosique, débarrassée ou non de la fraction hémicellulosique hydrolysée et le cas échéant de la lignine, est hydrolysée par les enzymes cellulolytiques et/ou hémicellulolytiques produites par les souches spécialisées, les cellulases de *Trichoderma reesei* étant les plus efficaces et les plus appropriées lorsque les substrats carbonés sont issus de la biomasse cellulosique ou ligno-cellulosique. Lesdites enzymes sont produites en utilisant au moins un effluent issu d'un autre procédé de production d'éthanol tel que décrit précédemment.

Le matériau à hydrolyser est mis en suspension en phase aqueuse à raison de 6 à 40 % de matière sèche, de préférence 20 à 30 %. Le pH est ajusté entre 4 et 5,5, de préférence entre 4,8 et 5,2 et la température entre 40 et 60°C, de préférence entre 45 et 50°C. La réaction d'hydrolyse est démarrée par ajout des cellulases ; la quantité habituellement utilisée est de 10 à 30 mg de protéines excrétées par gramme de substrat prétraité ou moins. La réaction dure généralement de 15 à 48 heures selon l'efficacité du prétraitement, la composition du mélange de cellulases et la quantité d'enzymes ajoutées. La réaction est suivie par dosage des sucres libérés, notamment le glucose. La solution sucrée est séparée de la fraction solide non hydrolysée, essentiellement constituée de lignine, par filtration ou centrifugation ; elle est utilisée pour la fermentation éthanolique.

En général, l'alcool est séparé du moût de fermentation par distillation et le résidu est constitué des vinasses de distillation. Ces vinasses riches en inducteur peuvent être utilisées comme source de carbone inductrice pour la production d'enzymes cellulolytiques et/ou hémicellulolytiques comme décrit dans la demande de brevet FR-A-2 881 753.

Les souches utilisées pour la production d'enzymes cellulolytiques et/ou hémicellulolytiques sont des souches de champignons appartenant aux genres *Trichoderma, Aspergillus, Penicillium* ou *Schizophyllum,* de préférence *Trichoderma reesei.* Toutefois, certains des champignons appartenant à ces genres, notamment *Trichoderma reesei,* ne peuvent utiliser le saccharose comme source de carbone. Il est donc nécessaire d'avoir recours à des souches génétiquement modifiées, par exemple exprimant de l'invertase de A. *niger,* comme décrit pour *Trichoderma reesei* par Berges et coll, Curr Genet. 1993 Jul-Aug 24 (1-2) : 53-59, afin que le saccharose puisse être utilisé comme source de carbone. Indépendamment, les souches peuvent être modifiées pour améliorer les enzymes cellulolytiques et/ou hémicellulolytiques par les procédés de mutation-sélection, comme par exemple la souche IFP CL847 (brevet français FR-B-2 555 803) ; les souches améliorées par les techniques de recombinaison génétique peuvent être également utilisées.

Ces souches sont cultivées en fermenteurs agités et aérés dans des conditions compatibles avec leur croissance et la production des enzymes. Selon sa nature, le substrat carboné choisi pour l'obtention de la biomasse est introduit dans le fermenteur avant stérilisation ou est stérilisé séparément et introduit dans le fermenteur après stérilisation de ce dernier pour avoir une concentration initiale de 20 à 35 g.L⁻¹. La source inductrice peut ne pas être ajoutée dans cette phase. Une solution aqueuse contenant le substrat choisi pour la production des enzymes est préparée à la concentration de 200-250 g.L⁻¹ ; cette solution doit contenir le substrat inducteur. Elle est injectée après l'épuisement du substrat initial de façon à apporter une quantité optimisée, comprise entre 35 et 45 mg.g⁻¹ de cellules ("fed batch"). La concentration résiduelle en sucre dans le milieu de culture est inférieure à 1 g.L⁻¹ pendant cette phase de "fed batch".

L'étape d'hydrolyse enzymatique est suivie d'une étape de fermentation, puis d'une étape de distillation et de séparation de l'alcool obtenu.

Dans le cas de la fermentation éthanolique, l'alcool obtenu est l'éthanol.

Dans le cas d'une fermentation de type acétonobutylique ou ABE, on obtient en fin de procédé un mélange d'alcools constitué de butanol et d'éthanol. Ce type de fermentation est réalisé par des bactéries appartenant au genre *Clostridium* et plus spécifiquement à l'espèce *Clostridium acetobutylicum.*

De la même façon, si la fermentation est du type IBE mettant en oeuvre les souches *Clostridium bejerinckii,* on obtient un mélange constitué d'isopropanol, de butanol et d'éthanol.

L'étape d'hydrolyse enzymatique et de fermentation peuvent être réalisées simultanément (procédé SSF pour Simultaneous Saccharification and Fermentation), puis sont suivies d'une étape de distillation et de séparation de l'alcool obtenu.

L'exemple 1 est donné à titre comparatif, et l'exemple 2 illustre l'invention, sans en limiter la portée.

### Exemple 1 : Production d'enzymes sur lactose

La production de cellulases est effectuée en fermenteur agité mécaniquement. Le milieu a la composition suivante: KOH 1,66 g.L⁻¹, H₃PO₄ 85 % 2 mL.L⁻¹, (NH4)₂SO₄ 2,8 g.L⁻¹, MgSO₄, 7 H₂O 0,6 g.L⁻¹, CaCL₂ 0,6 g.L⁻¹, MnSO₄ 3,2 mg.L⁻¹, ZnSO₄, 7 H₂O 2,8 mg.L⁻¹, CoCl₂ 4,0 mg.L⁻¹, FeSO₄, 7 H₂O 10 mg.L⁻¹, Corn Steep 1,2 g.L⁻¹, anti-mousse 0,5 mL.L⁻¹.

Le fermenteur contenant 1,75 L de milieu minéral et 70 g de lactose est stérilisé à 120 °C, puis ensemencé avec 0,25 L d'une préculture liquide de la souche de *Trichoderma reesei* CL847. Le milieu de la préculture, additionné de phtalate de potassium à 5 g.L⁻¹ pour contrôler les variations de pH, est identique à celui du fermenteur. La croissance du champignon en préculture est faite sur lactose, à la concentration de 30 g.L⁻¹. La croissance de l'inoculum dure de 2 à 3 jours et est effectuée entre 27 et 30 °C sur une table d'agitation.

Après 46 heures de croissance, le substrat initial du fermenteur est épuisé et la solution de lactose à 250 g.L⁻¹ est injectée en continu au débit de 4,5 mL.h⁻¹ jusqu'à 142 heures.

La température est régulée à 27°C pendant la phase de croissance de la biomasse, puis à 25°C jusqu'à la fin de la culture. Le pH est régulé à 5 pendant la phase de croissance, puis à 4 jusqu'à la fin de la culture par addition d'une solution d'ammoniaque qui apporte l'azote nécessaire à la synthèse des protéines excrétées. La teneur en oxygène dissous est maintenue au-dessus de 15 à 20 % par action sur l'aération et l'agitation.

La production d'enzymes est suivie par le dosage des protéines extracellulaires par la méthode de Folin (Lowry), après séparation du mycélium par filtration ou centrifugation. Les activités cellulolytiques sont déterminées par la méthode de l'activité papier filtre (UPF : unité papier filtre) pour une activité globale et l'activité cellobiase, activité considérée comme limitante du processus d'hydrolyse enzymatique de la biomasse lignocellulosique. L'activité UPF est mesurée sur papier Whatman n° 1 à la concentration initiale de 50 g.L⁻¹ ; on détermine la prise d'essai de la solution enzymatique à analyser qui libère l'équivalent de 2 g.L⁻¹ de glucose (dosage colorimétrique) en 60 minutes. L'activité cellobiase est mesurée sur cellobiose à la concentration de 20 mM ; on détermine la prise d'essai qui libère 0,5 g.L⁻¹ de glucose (dosage enzymatique) en 30 minutes.

Les activités en U.mL⁻¹ sont exprimées en micromoles de glucose libérées par minute et par millilitre de solution enzymatique.

Les déterminations analytiques sur le moût final donnent les résultats suivants :

| | |
|---|---|
| Substrat consommé g.L⁻¹ | 79,6 |
| Biomasse g.L⁻¹ | 13,5 |
| Protéines mg.mL⁻¹ | 37,8 |
| UPF U.mL⁻¹ | 22,1 |
| Cellobiases U.mL⁻¹ | 25,2 |

### Exemple 2 : Production d'enzymes sur Saccharose

La production d'enzymes est menée dans les mêmes conditions que dans l'Exemple 1, mais le lactose est remplacé dans la phase batch par du saccharose et le fed-batch réalisé avec une solution de 60% lactose 40% saccharose. La souche utilisée est une souche dérivée de CL847 transformée avec l'invertase de A. *niger* (Bergès et al, 1993).

Le fermenteur contenant 1,75 L de milieu minéral avec 40 g de saccharose pur est ensemencé avec 0,25 litre d'une préculture liquide de la souche de *Trichoderma reesei* CL847. Le substrat carboné de la préculture est le glucose à la concentration de 20 g.L⁻¹ Après 48 heures de croissance, après l'épuisement du substrat initial, la solution de lactose 60% et 40% saccharose à 200 g.L⁻¹ est injectée en continu au débit de 5 mL.h⁻¹ jusqu'à 165 heures.

Les déterminations analytiques sur le moût final donnent les résultats suivants :

| | |
|---|---|
| Substrat consommé g.L⁻¹ | 71.9 |
| Biomasse g.L⁻¹ | 12.2 |
| Protéines mg.mL⁻¹ | 32 |
| UPF U.mL⁻¹ | 23 |
| Cellobiases U.mL⁻¹ | 34 |

Les productions d'enzymes obtenues sont proches en termes de rendement et d'activités enzymatiques. Ces valeurs sont compatibles avec la réalisation d'une hydrolyse enzymatique de biomasse lignocellulosique efficace. Cette hydrolyse enzymatique et la fermentation donneront pour les deux exemples des résultats similaires en terme de production d'alcools.

## Revendications

1. Procédé de production d'alcool à partir de biomasse lignocellulosique prétraitée dans lequel l'étape d'hydrolyse enzymatique est réalisée avec des enzymes cellulolytiques et/ou hémicellulolytiques produites en utilisant au moins un effluent issu d'un autre procédé de production d'éthanol utilisant comme matière première une plante sucrière, ledit effluent comprenant des jus sucrés issus des lavages de betteraves ou de cannes à sucre, et/ou des mélasses de sucrerie, la production d'enzymes étant réalisée avec des souches de microorganisme cellulolytique appartenant aux genres *Trichoderma, Aspergillus, Penicillum ou Schizophyllumen,* utilisant le saccharose comme source de carbone pour sa croissance, naturellement ou après modification.

2. Procédé selon la revendication 1 dans lequel la production d'enzymes cellulolytiques et/ou hémicellulolytiques est réalisée avec des champignons utilisant en outre comme source de carbone inductrice un autre sucre soluble industriel ou un extrait de la fraction hémicellulosique sous forme de monomères provenant de la biomasse prétraitée ou un extrait de vinasses de fermentation de produits d'hydrolyse de biomasse lignocellulosique ou un mélange de ceux -ci.

3. Procédé selon l'une des revendications 1 ou 2 dans lequel le microorganisme appartient à l'espèce *Trichoderma reesei.*

4. Procédé selon l'une des revendications précédentes dans lequel la biomasse lignocellulosique est choisie parmi les pailles, le bois, les cultures forestières, les résidus de plantes alcooligènes, sucrières et céréalières, les résidus de l'industrie papetière et les produits de transformation des matériaux cellulosiques et lignocellulosiques.

5. Procédé selon l'une des revendications précédentes dans lequel l'étape d'hydrolyse enzymatique est suivie d'une étape de fermentation, puis d'une étape de distillation et de séparation de l'alcool.

6. Procédé selon la revendication 5 dans lequel l'étape de fermentation est de type fermentation éthanolique et l'alcool obtenu est l'éthanol.

7. Procédé selon la revendication 5 dans lequel l'étape de fermentation est de type fermentation ABE et l'alcool obtenu est un mélange de butanol et d'éthanol.

8. Procédé selon la revendication 5 dans lequel l'étape de fermentation est de type fermentation IBE et l'alcool obtenu est un mélange de butanol, d'éthanol et d'isopropanol.

9. Procédé selon l'une des revendications 1 à 8 dans lequel l'hydrolyse enzymatique et la fermentation sont réalisées simultanément, et sont suivies d'une étape de distillation et de séparation de l'alcool.

## Claims

1. A method of producing alcohol from pretreated lignocellulosic biomass, wherein the enzymatic hydrolysis stage is carried out with cellulolytic and/or hemicellulolytic enzymes produced using at least one effluent from another ethanol production process using a sugar plant as the feedstock, said effluent used for the production of cellulolytic and/or hemicellulolytic enzymes comprising sweet juices resulting from beet or sugar cane washing, and/or sugar refinery molasses, the enzyme production being made with microorganism strains belonging to the *Trichoderma, Aspergillus, Penicillium* or *Schizophyllum* genera, using saccharose as the carbon source for its growth, naturally or after modification.

2. A method as claimed in claim 1, wherein the production of cellulolytic and/or hemicellulolytic enzymes is achieved with fungi also using as the carbon source another industrial soluble sugar or a hemicellulosic fraction extract in form of monomers resulting from the pretreated biomass, or a fermentation vinasse extract from lignocellulosic biomass hydrolysis products, or a mixture thereof.

3. A method as claimed in claim 1 or 2, wherein the microorganism belongs to the *Trichoderma reesei* species.

4. A method as claimed in any one of the previous claims, wherein the lignocellulosic biomass is selected from among straws, wood, forest crops, alcohol-producing, sugar crop and cereal crop residues, paper industry residues, cellulosic and lignocellulosic material transformation products.

5. A method as claimed in any one of the previous claims, wherein the enzymatic hydrolysis stage is followed by a fermentation stage, then by a stage of distillation and separation of the alcohol,

6. A method as claimed in claim 5, wherein the fermentation stage is of ethanolic fermentation type and the alcohol obtained is ethanol.

7. A method as claimed in claim 5, wherein the fermentation stage is of ABE fermentation type and the alcohol obtained is a mixture of butanol and ethanol.

8. A method as claimed in claim 5, wherein the fermentation stage is of IBE fermentation type and the alcohol obtained is a mixture of butanol, ethanol and isopropanol.

9. A method as claimed in any one of claims 1 to 8, wherein enzymatic hydrolysis and fermentation are carried out simultaneously and are followed by a stage of distillation and separation of the alcohol.

## Patentansprüche

1. Verfahren zur Herstellung von Alkohol aus vorbehandelter lignozellulosehaltiger Biomasse, wobei der Schritt der enzymatischen Hydrolyse mit zellulosespaltenden und/oder hemizellulosespaltenden Enzymen durchgeführt wird, die unter Verwendung mindestens eines Abflusses aus einem Verfahren zur Herstellung von Ethanol unter Verwendung einer Zuckerpflanze als Ausgangsmaterial hergestellt werden, wobei der Abfluss zuckerhaltige Säfte umfasst, die aus der Zuckerrüben- oder Zuckerrohrwäsche und/oder aus Zuckerraffineriemelassen stammen, wobei die Enzymherstellung mit Stämmen zellulosespaltender Mikroorganismen durchgeführt wird, die zu den Gattungen *Trichoderma*, *Aspergillus*, *Penicillum* oder *Schizophyllum* gehören, wobei natürliche oder modifizierte Saccharose als Kohlenstoffquelle für ihr wachstum verwendet wird.

2. Verfahren nach Anspruch 1, wobei die Herstellung zellulosespaltender und/oder hemizellulosespaltender Enzyme mit Pilzen durchgeführt wird, bei der als induzierende Kohlenstoffquelle außerdem ein anderer löslicher Industriezucker oder ein Extrakt der hemizellulosehaltigen Fraktion in Form von Monomeren verwendet wird, der aus der vorbehandelten Biomasse oder aus einem Melasseschlempenextrakt aus der Fermentation der Hydrolyseprodukte der lignozellulosehaltigen Biomasse oder einem Gemisch davon stammt.

3. Verfahren nach einem der Ansprüche 1 oder 2, wobei der Mikroorganismus zur Spezies *Trichoderma reesei* gehört.

4. Verfahren nach einem der vorhergehenden Ansprüche, wobei die lignozellulosehaltige Biomasse ausgewählt ist aus Stroh, Holz, Forstkulturen, Rückständen von alkoholproduzierenden Pflanzen, Zuckerpflanzen und Getreidepflanzen, Rückständen der Papierindustrie und Transformationsprodukten der zellulosehaltigen und lignozellulosehaltigen Materialien.

5. Verfahren nach einem der vorhergehenden Ansprüche, wobei auf den Schritt der enzymatischen Hydrolyse ein Schritt zur Fermentation, dann ein Schritt zur Destillation und zur Trennung des Alkohols folgt.

6. Verfahren nach Anspruch 5, wobei es sich bei dem Schritt zur Fermentation um eine ethanolische Fermentation handelt und der erhaltene Alkohol Ethanol ist.

7. Verfahren nach Anspruch 5, wobei es sich bei dem Schritt zur Fermentation um eine ABE-Fermentation handelt und der erhaltene Alkohol ein Gemisch aus Butanol und Ethanol ist.

8. Verfahren nach Anspruch 5, wobei es sich bei dem Schritt zur Fermentation um eine IBE-Fermentation handelt und der erhaltene Alkohol ein Gemisch aus Butanol, Ethanol und Isopropanol ist.

9. Verfahren nach einem der Ansprüche 1 bis 8, wobei die enzymatische Hydrolyse und die Fermentation gleichzeitig durchgeführt werden und darauf ein Schritt zur Destillation und zur Trennung des Alkohols folgt.
